# FASCICULE DE BREVET EUROPEEN

(11) **EP 1 345 577 B1**
(45) Date de publication et mention de la délivrance du brevet: **13.02.2008**
(21) Numéro de dépôt: 01994911.4
(22) Date de dépôt: 19.12.2001
(51) Int. Cl.: A61K 8/81, A61K 8/41, A61Q 5/10

(54) **COMPOSITION DE TEINTURE D'OXYDATION POUR FIBRES KERATINIQUES A BASE DE POLYMERES AMPHIPHILIES D'AU MOINS UN MONOMERE A INSATURATION ETHYLENIQUE A GROUPEMENT SULFONIQUE ET COMPORTANT UNE PARTIE HYDROPHOBE**
OXIDATIONSFÄRBEZUSAMMENSETZUNG FÜR KERATINÖSE FASERN AUF BASIS VON AMPHIPHILEN POLYMEREN ZUMINDEST EINES ETHYLENISCH UNGESÄTTIGTEN MONOMERS MIT SULFONGRUPPE UND EINEM HYDROPHOBEN TEIL
OXIDATION DYEING COMPOSITION FOR KERATINOUS FIBRES BASED ON AMPHIPHILIC POLYMERS OF AT LEAST AN ETHYLENICALLY UNSATURATED MONOMER WITH SULPHONIC GROUP AND COMPRISING A HYDROPHOBIC PART

(30) Priorité: 22.12.2000 FR 0016949; 11.01.2001 FR 0100326
(43) Date de publication de la demande: 24.09.2003
(73) Titulaire: L'ORÉAL, 75008 Paris (FR)
(72) Inventeur: KRAVTCHENKO, Sylvain, F-92600 Asnieres (FR); LAGRANGE, Alain, F-77770 Coupvray (FR)
(74) Mandataire: Wattremez, Catherine
(86) Numéro de dépôt international: PCT/FR2001/004075
(87) Numéro de publication internationale: WO 2002/051367

(56) Documents cités:
- EP-A- 0 406 042
- EP-A- 0 750 899
- EP-A- 0 815 843
- EP-A- 0 823 250
- EP-A- 0 970 685
- EP-A- 1 055 406
- WO-A-00/31154
- US-A- 4 460 732
- US-A- 4 861 499
- US-A- 5 089 578
- US-A- 5 114 706
- US-A- 5 464 452
- US-A- 5 932 201
- A KABAYASHI ET ALL: "Solubilization Properties of N-substituted Amphiphilic Acrylamide Copolymers" JOURNAL OF APPLIED POLYMER SCIENCE., vol. 73, no. 12, 19 septembre 1999 (1999-09-19), pages 2447-2453, XP002177425 JOHN WILEY AND SONS INC. NEW YORK., US ISSN: 0021-8995
- PATENT ABSTRACTS OF JAPAN vol. 1997, no. 02, 28 février 1997 (1997-02-28) & JP 08 252447 A (SHISEIDO CO LTD), 1 octobre 1996 (1996-10-01)

## Description

La présente invention concerne une composition de teinture d'oxydation des fibres kératiniques, en particulier des fibres kératiniques humaines et plus particulièrement des cheveux, comprenant au moins un colorant d'oxydation et au moins un polymère amphiphile comportant au moins un monomère à insaturation éthylénique à groupement sulfonique, sous forme libre ou partiellement ou totalement neutralisée et en outre au moins une partie hydrophobe.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para-phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Pour localiser le produit de coloration d'oxydation à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, certains polyuréthanes, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les systèmes épaississants mentionnés ci-dessus ne permettaient pas d'obtenir des nuances puissantes et chromatiques de faibles sélectivités et de bonnes ténacités tout en assurant un bon état cosmétique à la chevelure traitée. Par ailleurs, elle a également constaté que les compositions tinctoriales prêtes à l'emploi contenant le ou les colorants d'oxydation, et les systèmes épaississants de l'art antérieur ne permettaient pas une application suffisamment précise sans coulures ni chutes de viscosité dans le temps.

Or, après d'importantes recherches menées sur la question, la Demanderesse vient maintenant de découvrir qu'il est possible d'obtenir des compositions de teinture d'oxydation prêtes à l'emploi qui ne coulent pas et restent donc bien localisées au point d'application, et qui permettent aussi d'obtenir des nuances puissantes et chromatiques (lumineuses) avec de faibles sélectivités et de bonnes ténacités vis à vis des agents chimiques ( shampooing, permanentes ...) ou naturels (lumière, transpiration ...) tout en apportant aux cheveux de bonnes propriétés cosmétiques, si on introduit (i) soit dans la composition contenant le ou les colorants d'oxydation et éventuellement le ou les coupleurs [ou composition A], soit (ii) dans la composition oxydante [ou composition B], ou (iii) dans les deux compositions à la fois, une quantité efficace d'un polymère amphiphile comportant au moins un monomère à insaturation éthylénique à groupement sulfonique, sous forme libre ou partiellement ou totalement neutralisée et en outre au moins une partie hydrophobe.
La demanderesse a constaté en outre que les mélanges des compositions colorantes avec les oxydants sont facilités par la présence des polymères amphiphiles selon l'invention.
Ces découvertes sont à la base de la présente invention.

La présente invention a ainsi pour objet une composition de teinture d'oxydation pour fibres kératiniques, en particulier pour fibres kératiniques humaines, et en particulier les cheveux, comprenant, dans un milieu approprié pour la teinture, au moins un colorant d'oxydation, qui est caractérisée par le fait qu'elle comprend en outre au moins un polymère amphiphile comportant au moins un monomère à insaturation éthylénique à groupement sulfonique, sous forme libre ou partiellement ou totalement neutralisée et en outre au moins une partie hydrophobe.

Un autre objet de l'invention porte sur une composition prête à l'emploi pour la teinture des fibres kératiniques qui comprend au moins un colorant d'oxydation, au moins un agent oxydant et au moins un polymère amphiphile comportant au moins un monomère à insaturation éthylénique à groupement sulfonique, sous forme libre ou partiellement ou totalement neutralisée et en outre au moins une partie hydrophobe.
Par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

L'invention vise également un procédé de teinture des fibres kératiniques, et en particulier des fibres kératiniques humaines telles que les cheveux, consistant à appliquer sur les fibres au moins une composition colorante comprenant, dans un milieu approprié pour la teinture, au moins un colorant d'oxydation, la couleur étant révélée à pH alcalin, neutre ou acide, à l'aide d'une composition oxydante comprenant au moins un agent oxydant qui est mélangée juste au moment de l'emploi à la composition colorante ou qui est appliquée séquentiellement sans rinçage intermédiaire, au moins un polymère amphiphile comportant au moins un monomère à insaturation éthylénique à groupement sulfonique, sous forme libre ou partiellement ou totalement neutralisée et en outre au moins une partie hydrophobe, étant présent dans la composition colorante ou dans la composition oxydante ou dans chacune des deux compositions.

L'invention a également pour objet des dispositifs de teinture ou « kits » à plusieurs compartiments.
Un dispositif à 2 compartiments selon l'invention comprend un compartiment- renfermant une composition colorante comprenant, dans un milieu approprié pour la teinture, au moins un colorant d'oxydation, et un autre compartiment renfermant une composition oxydante comprenant, dans un milieu approprié pour la teinture, un agent oxydant, le polymère amphiphile selon l'invention étant présent dans la composition colorante ou la composition oxydante, ou dans chacune des deux compositions.

Un autre dispositif, à 3 compartiments selon l'invention, comprend un premier compartiment renfermant une composition colorante comprenant, dans un milieu approprié pour la teinture, au moins un colorant d'oxydation, un deuxième compartiment renfermant une composition oxydante comprenant, dans un milieu approprié pour la teinture au moins un agent oxydant, et un troisième compartiment renfermant une composition comprenant, dans un milieu approprié pour la teinture, au moins un polymère amphiphile selon l'invention décrit ci-dessus, la composition colorante et/ou la composition oxydante pouvant également comprendrer un polymère amphiphile selon l'invention.

Mais d'autres caractéristiques, aspects, objets et avantages de l'invention apparaîtront encore plus clairement à la lecture de la description et des exemples qui suivent.

### Polymères amphiphiles selon l'invention

Les polymères conformes à l'invention sont des polymères amphiphiles comportant au moins un monomère à insaturation éthylènique à groupement sulfonique qui est l'acide 2-acrylamido-2-méthylpropane-sulfonique, sous forme libre ou partiellement ou totalement neutralisée et comprenant au moins une partie hydrophobe.
On entend par polymère amphiphile, tout polymère comportant à la fois une partie hydrophile et une partie hydrophobe et notamment une chaîne grasse.

a partie hydrophobe présente dans les polymères de l'invention comporte de 6 à 50 atomes de carbone, plus préférentiellement de 6 à 22 atomes de carbone, plus préférentiellement encore de 6 à 18 atomes de carbone, et plus particulièrement de 12 à 18 atomes de carbone.

De façon préférentielle, les polymères conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme farginine et la lysine, et les mélanges de ces composés.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés.
De préférence, on choisit des polymères amphiphiles réticulés.

Lorsqu'ils sont réticulés, les agents de réticulation peuvent être choisis parmi les composés à polyinsaturation oléfinique couramment utilisés pour la réticulation des polymères obtenus par polymérisation radicalaire.
On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les polymères amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154 (faisant partie Intégrante du contenu de la description). Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères de l'invention sont choisis parmi les copolymères amphiphiles d'AMPS et d'au moins un monomère hydrophobe à insaturation éthylénique comportant au moins une partie hydrophobe ayant de 6 à 50 atomes de carbone et plus préférentiellement de 6 à 22 atomes de carbone et encore plus préférentiellement de 6 à 18 atomes de carbone et plus particulièrement 12 à 18 atomes de carbone.

Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N°10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les monomères hydrophobes à insaturation éthylénique des copolymères de l'invention sont choisis parmi les acrylates ou les acrylamides de formule (I) suivante : 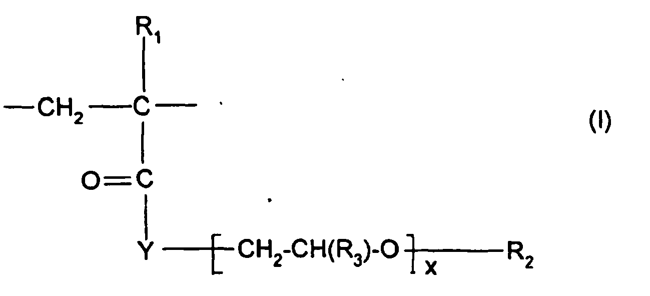 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant au moins de 6 à 50 atomes de carbone et plus préférentiellement de 6 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (I) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1, à 10% en mole de motifs AMPS et de 5 à 80% en mole de motifs n-(C₆-C₁₈)alkylacrytamide, tels que ceux décrits dans le brevet US- 5089578.

On peut également citer les copolymères d'AMPS totalement neutralisé et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (II) suivante : 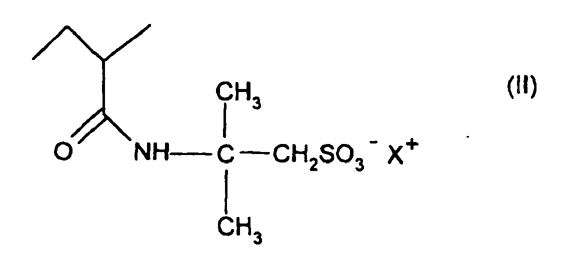 dans laquelle X⁺ est un proton, un cation de métal alcalin, un cation alcalino-terreux ou l'ion ammonium,
et de motifs de formule (III) suivante : 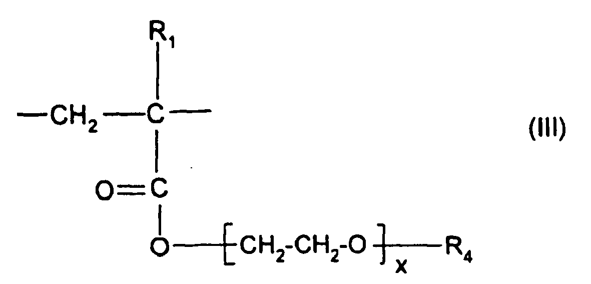 dans laquelle x désigne un nombre entier variant de 3 à 100, de préférence de 5 à 80 et plus préférentiellement de 7 à 25 ; R₁ a la même signification que celle indiquée ci-dessus dans la formule (1) et R₄ désigne un alkyle linéaire ou ramifié en C₆-C₂₂ et plus préférentiellement en C₁₀-C₂₂.

Les polymères particulièrement préférés sont ceux pour lesquels x = 25 , R₁ désigne méthyle et R₄ représente n-dodécyle ; ils sont décrits dans les articles de Morishima mentionnés ci-dessus.

Les polymères pour lesquels X⁺ désigne sodium ou ammonium sont plus particulièrement préférés.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH ( 2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.

En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image.

Une distribution intéressante pour de type de polymère et déterminée par analyse d'image est la suivante: 60,2% inférieur à 423 microns, 52.0% inférieur à 212 microns, 26,6% inférieur à 106 microns, 2,6% inférieur à 45 microns et 26,6% supérieur à 850 microns.

La réaction peut être conduite à une température comprise entre 0 et 150°C, de préférence entre 10 et 100°C , soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL® C-080 de la société HOECHSTICLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHSTICLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHSTICLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL® T-150 de la société HOECHST/CLARIANT),
- d'un alcool en C₁₆-C₁₈ oxyéthyléné par 11 moles d'oxyde d'éthylène (GENAPOL® T-110 de la société HOECHST/CLARIANT),
- d'un alcool en C₁₆-C₁₈ oxyéthyléné par 20 moles d'oxyde d'éthylène (GENAPOL^{®} T-200 de la société HOECHSTICLARIANT),
- d'un alcool en C₁₆-C₁₈ oxyéthyléné par 25 moles d'oxyde d'éthylène (GENAPOL^{®} T-250 de la société HOECHST/CIARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier de 0,1 à 99,9% en moles.

De préférence pour les polymères les plus hydrophobes, la proportion molaire en motifs de formule (I) ou (III) varie de 50,1 à 99,9%, plus particulièrement de 70 à 95% et encore plus particulièrement de 80 à 90%.

De préférence pour les polymères peu hydrophobes, la proportion molaire en motifs de formule (I) ou (III) varie de 0,1 à 50%, plus particulièrement de 5 à 25% et encore plus particulièrement de 10 à 20%.

La distribution des monomères dans les polymères de l'invention peut être, par exemple, alternée, bloc (y compris multibloc) ou quelconque.

Selon l'invention, on préfère que les polymères aient des chaînes pendantes sensibles à la chaleur et dont la solution aqueuse présente une viscosité qui, au delà d'une certaine température seuil, augmente, ou demeure pratiquement constante quand la température augmente.

Plus particulièrement encore, on préfère les polymères dont la solution aqueuse présente une viscosité qui est faible en dessous d'une première température seuil et qui, au dessus de cette première température seuil croît vers un maximum quand la température augmente, et qui, au dessus d'une seconde température seuil décroît à nouveau quand la température augmente. Dans cet optique, on préfère que la viscosité des solutions de polymère en dessous de la première température seuil soit de 5 à 50%, en particulier de 10 à 30% de la viscosité maximum à la seconde température seuil.

Ces polymères, de préférence, conduisent dans l'eau à un phénomène de démixion par chauffage se traduisant par des courbes présentant, en fonction de la température et de la concentration, un minimum appelé LCST (Lower Critical Solution Temperature).

Les viscosités (mesurées à 25°C au viscosimètre Brookfield aiguille 7) des solutions aqueuses à 1% vont de préférence de 20 000 mPas à 100 000 mPas et plus particulièrement de 60 000 mPas à 70 000 mPas.

Les polymères amphiphiles conformes à l'invention sont présents dans les compositions dans des concentrations allant de 0,01 à 30% en poids, plus préférentiellement de 0,1 à 10%, encore plus préférentiellement de 0,1 à 5% en poids et plus particulièrement encore de 0,5 à 2% en poids.

### Colorants d'oxydation

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.

On peut notamment citer :
- (I) les paraphénylènediamines de formule (I) suivante et leurs sels d'addition avec un acide : 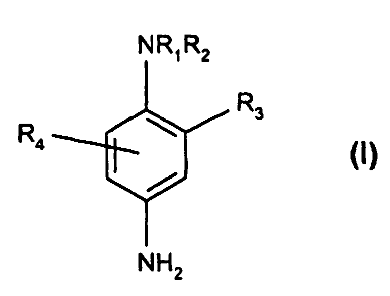 dans laquelle:
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄:

   Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)aMino, imidazolinium et ammonium.
   Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxjréthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamirie, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthytoxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (II) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (II) suivante, et leurs sels d'addition avec un acide : 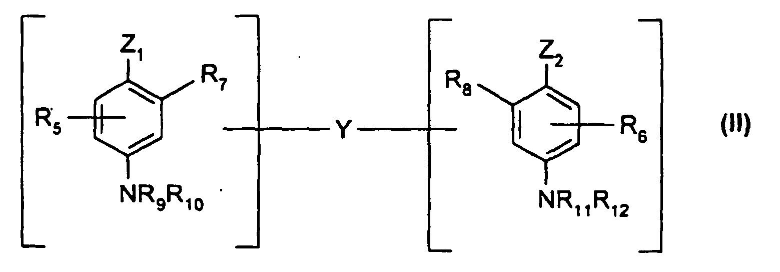 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N.N'-bis-(β-hydroxyéthyl)-N,N'-bis-(4'-aminophényl)-éthylènediamine, la N,N'-bis-(4-aminophényl)-tétraméthylènediamine, la N,N'=bis-(β-hydroxyéthyl)-N,N'-bis-(4-aminophényl)-tétraméthylènediamine, la N,N'-bis-(4-méthyl-aminophényl)-tétraméthylènediamine, la N, N'-bis-(éthyl)-N, N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (III) les para-aminophénols répondant à la formule (III) suivante, et leurs sels d'addition avec un acide : 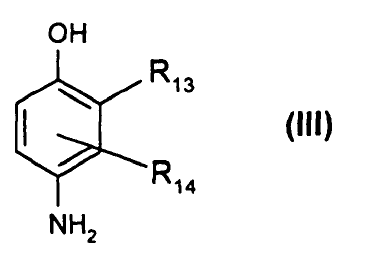 dans laquelle:
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hy,droxyméthyl-phénol, le 4-arnino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765. comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyt pyrazole, le 4,5-diarnino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 2-méthyl-3-arnino-6-méthyl-phénol, la 3,5-diamino-2,6-diméthoxypyridine, la 6-hydroxybenzomorpholine, le 1-β-hydroxyéthylamino-3,4-méthylènedioxy-benzène, le 1-méthyl-2,6-bis(β-hydroxyéthylamino)-benzène, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide. Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore comprendre, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition colorante et/ou la composition oxydante peuvent en outre plus particulièrement comprendre, au moins un polymère amphotère ou cationique.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 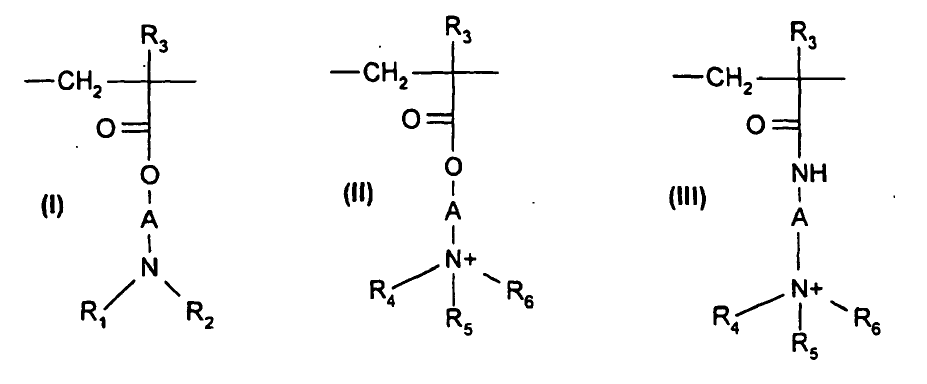 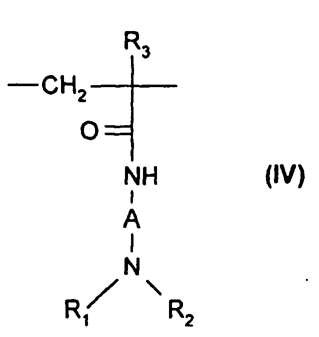 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthyl-ammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthyl-ammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP.
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyt triméthylammonium, de méthacrylmidopropyl triméthylammonium, de diméthyl-diallylammonium. Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropylldiéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 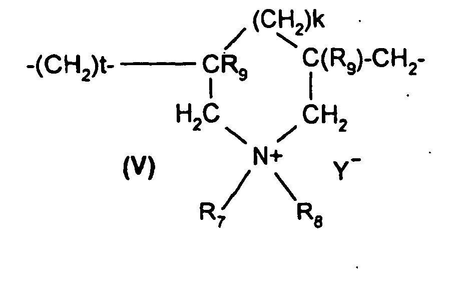 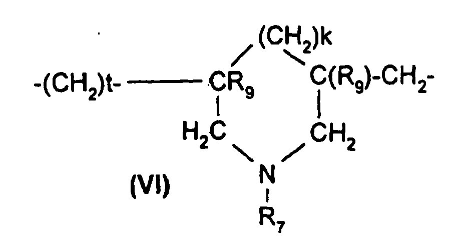 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 8 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 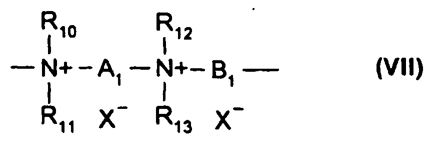 formule (VII) dans laquelle :
   R₁₀. R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)ₙ-CO-D-OC-(CH₂)ₙ- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂-;
      d) un groupement uréylène de formule : -NH-CO-NH-.

   De préférence, X- est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 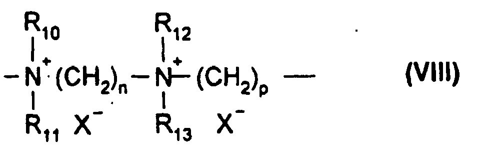 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs récurrents de formule (IX): 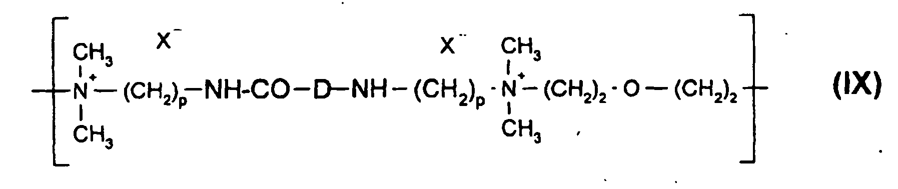 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de "POLYETHYLENEGLYCOL (15) TALLOW POLYAMINE "dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 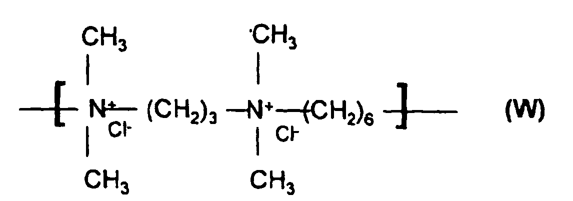 et notamment ceux dont le poids moléculaire, déterminé par chromatographie par perméation de gel, est compris entre 9500 et 9900; 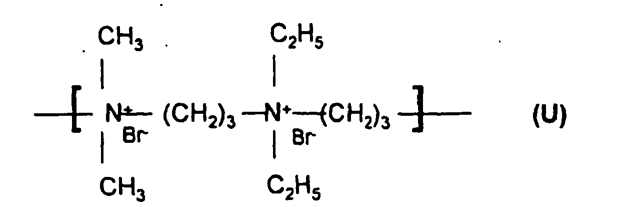 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamidelacrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 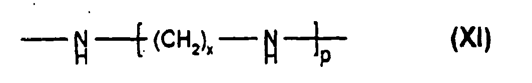 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylèhediamine, ou le radical dérivant de la pipérazine : 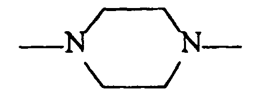
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule: 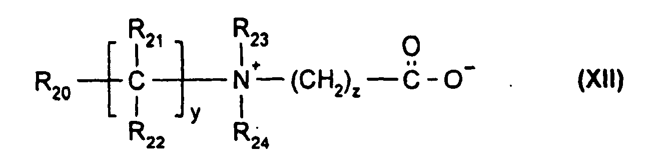 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylarnrnonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 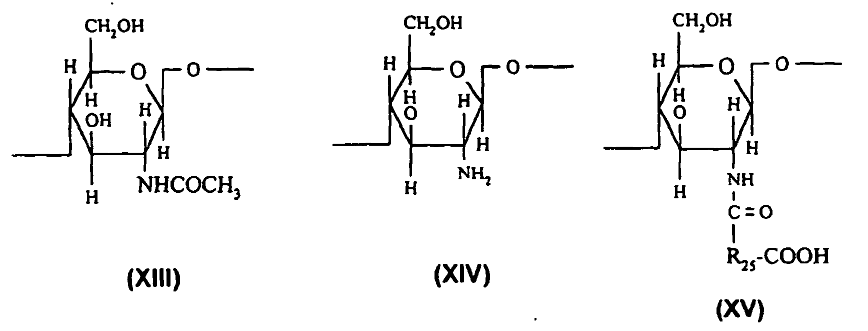 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 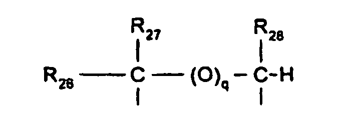 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits, par exemple dans le brevet français 1 400 366 : 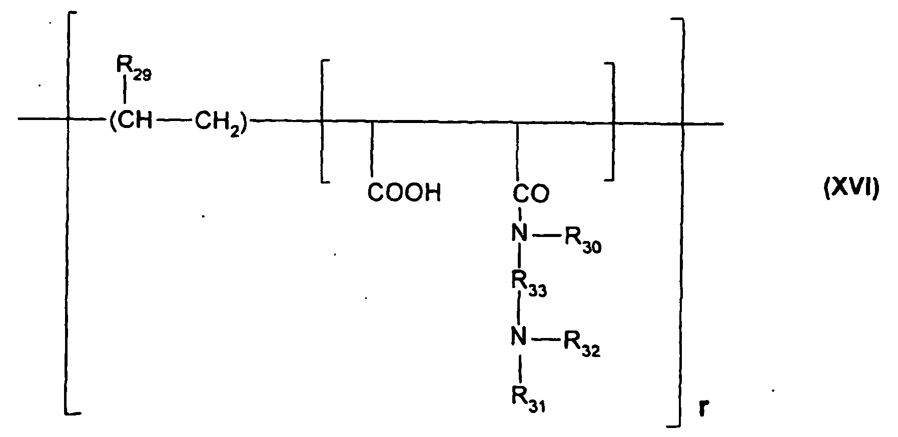 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂-, -CH₂-CH(CH₃)-, R₃₁ ayant les significations mentionnées ci-dessus, ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 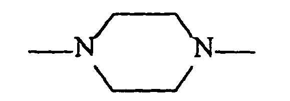 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule:

      -D-X-D-X- (XVIII)
   où D désigne un radical 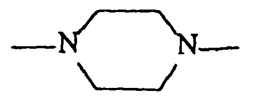 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs. Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les .condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide Rg -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylampho-dipropionate, Disodium Capryloamphodipropionate, Lauroamphodipropionic acid, Coco-amphodipropionic acid.

A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,1 à 30% du poids total de la composition.

Le milieu de la composition approprié pour la teinture, est de préférence un milieu aqueux constitué par de l'eau et peut avantageusement comprendre des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations allant d'environ 0,5 à 20% et, de préférence, d'environ 2 à 10% en poids par rapport au poids total de la composition.

La composition colorante peut encore comprendre une quantité efficace d'autres agents, par ailleurs antérieurement connus en coloration d'oxydation, tels que divers adjuvants usuels comme des agents séquestrants tels que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ladite composition peut également comprendre des agents réducteurs ou antioxydants. Ceux-ci peuvent être choisis en particulier parmi le sulfite de sodium, l'acide thioglycolique, l'acide thiolactique, le métabisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition oxydante, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement d'une valeur allant de 4 à 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 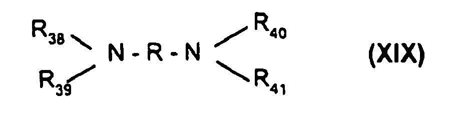 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions colorante et oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Une variante de ce procédé consiste à prendre une composition comprenant au moins un colorant d'oxydation mais sans polymère amphiphile selon l'invention, une autre composition comprenant au moins un polymère amphiphile, et à mélanger au moment de l'emploi ces deux compositions avec la composition oxydante, puis à appliquer et laisser agir le mélange obtenu comme précédemment.

Selon lesdits procédés, chacune de ces trois compositions ou les trois compositions peuvent comprendre en outre au moins un polymère cationique ou amphotère et au moins un tensio-actif.

Des exemples concrets illustrant l'invention sont indiqués ci-après, sans pour autant présenter un caractère limitatif.

### EXEMPLES DE PREPARATION:

Préparation des esters (méth)acryliques éthoxylés.

Ils peuvent être notamment obtenus par action de (méth)acrylate de glycidyle, ou d'acide (méth)acrylique, ou d'un (méth)acrylate d'alkyle, ou d'un halogénure de (méth)acryloyle sur un alcool gras éthoxylé. On peut citer, à titre d'exemples non limitatifs, les préparations suivantes :
a) à partir du méthacrylate de glycidyle et du GENAPOL T-250
b) à partir de l'acide (méth)acrylique et du GENAPOL UD-070
c) à partir du (méth)acrylate de méthyle et du GENAPOL LA-090
d) à partir du chlorure de (méth)acryloyle et du GENAPOL UD-070.

a) Dans un réacteur tricol d'un litre équipé d'un agitateur, d'un thermomètre et d'un condensateur à reflux, on introduit 500g de Genapol T-250 et 75g de méthacrylate de glycidyle. On chauffe le mélange réactionnel à la température de 100°C pendant 2 heures, et on élimine l'excès de méthacrylate de glycidyle par distillation sous pression réduite. Le monomère obtenu peut être utilisé pour la polymérisation sans purification ultérieure.
b) Dans un réacteur tricol d'un litre équipé d'un agitateur, d'un thermomètre et d'un condensateur à reflux, on introduit 500g de Genapol UD-070, 100g d'acide (méth)acrylique et de l'acide p-toluènesulfonique comme catalyseur. On chauffe le mélange réactionnel au reflux pendant 2 heures, et l'on sépare l'excès d'acide et d'eau formée au cours de la réaction par distillation sous pression réduite. Le monomère obtenu peut être utilisé pour la polymérisation sans purification ultérieure.
c) Dans un réacteur tricol d'un litre équipé d'un agitateur, d'un thermomètre et d'un condensateur à reflux, on introduit 500g de Genapol LA-090, 100g de (méth)acrylate de méthyle et 20g de tétraisopropoxyde de titane. On chauffe le mélange réactionnel au reflux pendant 2 heures, et après séparation par distillation de l'alcool formé, on distille l'ester qui reste sous pression réduite.
   Le monomère obtenu peut être utilisé pour la polymérisation sans purification ultérieure.
d) Dans un réacteur tricol d'un litre équipé d'un agitateur, d'un thermomètre et d'un condensateur à reflux, on introduit 500g de Genapol UD-070, 110g de chlorure de (méth)acryloyle et 50g de carbonate de sodium. On chauffe le mélange réactionnel au reflux pendant 2 heures, et l'on sépare l'excès de chlorure d'acide par distillation sous pression réduite. Le monomère obtenu peut être utilisé pour la polymérisation sans purification ultérieure.

### Polymérisation selon la méthode de précipitation dans le tert-butanol

Dans un réacteur de 2 litres muni d'un condensateur à reflux, d'une prise de gaz, d'un thermomètre et d'un agitateur, on introduit 500ml de tert-butanol, et la quantité calculée d'AMPS. On neutralise le mélange en introduisant du NH3, et on ajoute le monomère préparé ci-dessus au mélange réactionnel. On rend le mélange réactionnel inerte par passage d'azote ou d'argon et lorsque la température intérieure a atteint 60°C, on introduit l'initiateur (AIBN) pour amorcer la polymérisation.
Après quelques minutes, le polymère ainsi préparé précipite. On porte le mélange à reflux pendant 2 heures, et on sépare le polymère du solvant par filtration à la trompe, puis on le sèche sous pression réduite.

De la façon décrite ci-dessus, on a préparé les polymères suivants :
(à partir des réactants suivants en des quantités exprimées en grammes)

| | | | | |
|---|---|---|---|---|
| Méthacrylate de Genapol T-250 | 10 | 20 | 30 | 97 |
| AMPS neutralisé par NH3 | 90 | 80 | 90 | 3 |
| Méthylène-bis-acrylamide (réticulant) | | | 1,5 | |
| Méthacrylate d'allyle (réticulant) | | 1,7 | | |
| TMPTA (réticulant) | 1,8 | | | 1,8 |
| Azobisisobutyronitrile (initiateur) | | | 1 | |
| Peroxyde dilauryle (initiateur) | 1 | 1 | | 1 |
| Tert-butanol | 300 | 300 | 300 | 300 |

### EXEMPLES DE COMPOSITIONS DE TEINTURE:

On a préparé la composition suivante :

### (quantités exprimées en grammes)

### Composition oxydante:

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | q.s. |
| Eau déminéralisée qsp | 100 |

### Composition colorante :

| | |
|---|---|
| Copolymère acide acrylamido-2-méthyl-2-propane-sulfonique/n-dodécylacrylamide (3,5%196,5%) neutralisé à 100% par la soude | 1 |
| Alcool isostéarylique (TEGO ALKANOL 66 vendu par la société GOLDSCHMIDT) | 12 |
| Alcool benzylique | 4 |
| Polyéthylèneglycol à 8 moles d'oxyde d'éthylène | 3 |
| Ethanol | 10 |
| Paraphénylènediamine | 0,54 |
| 2-méthyl-5-amino-phénol | 0,615 |
| Métabisulfite de sodium | 0,2 |
| Agent séquestrant | q.s. |
| Ammoniaque à 20% d'NH3 | 10 |
| Eau déminéralisée q.s.p | 100 |

La composition colorante a été mélangée, au moment de l'emploi, dans un bol en plastique et pendant 2 minutes, à la composition oxydante donnée ci-dessus, à raison de 1 partie de composition colorante pour 1 partie de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing standard, à nouveau rincées à l'eau, puis séchées.
Les cheveux ont été teints dans une nuance violine uniforme.

Des résultats similaires ont été obtenus en remplaçant le copolymère acide acrylamido-2-méthyl-2-propane-sulfonique/n-dodécylacrylamide neutralisé par la soude de l'exemple ci-dessus par un copolymère réticulé par du méthylène-bis-acrylamide constitué de 75% en poids de motifs AMPS neutralisés par NH₃ et de 25% en poids de motifs de formule (III) dans laquelle R₁=H, R₄=C₁₆-C₁₈ et x=25 .

Des résultats similaires ont été obtenus en remplaçant le copolymère acide acrylamido-2-méthyl-2-propane-sulfonique/n-dodécylacrylamide neutralisé par la soude de l'exemple ci-dessus par un copolymère réticulé par du méthacrylate d'allyle constitué de 90% en poids de motifs AMPS neutralisés par NH₃ et de 10% en poids de motifs méthacrylate de GENAPOL T-250 [motifs de formule (III) dans laquelle R₁=CH₃, R₄=C₁₆-C₁₈ et x=25], ou par un copolymère réticulé par du méthacrylate d'allyle constitué de 80% en poids de motifs AMPS neutralisés par NH₃ et de 20% en poids de motifs méthacrylate de GENAPOL T-250 [motifs de formule (III) dans laquelle R₁=CH₃, R₄=C₁₆-C₁₈ et x=25].

## Revendications

1. Composition de teinture d'oxydation pour fibres kératiniques, en particulier pour fibres kératiniques humaines et plus particulièrement des cheveux, comprenant dans un milieu approprié pour la teinture, au moins un colorant d'oxydation, **caractérisée par le fait qu'**elle comprend en outre au moine un polymère amphiphile comportant au moins un monomère à insaturation éthylénique à groupement sulfonique qui est l'acide 2-acrylamido-2-méthylpropane-sulfonique sous forme libre ou partiellement ou totalement neutralisée et en outre au mains une partie hydrophobe comportant de 6 à 50 atomes de carbone et correspondant à au moins un monomère hydrophobe à insaturation éthylénique choisi parmi les acrylates ou les acrylamides de formule (I) suivante : 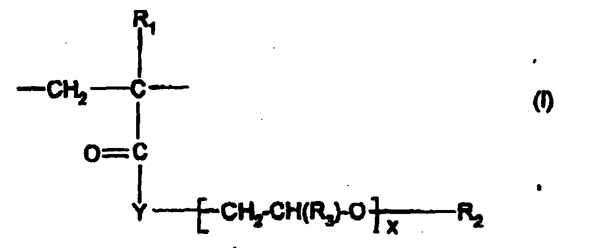 dans laquelle R₁ et R₃, identiques ou différents, désignent un atome d'hydrogène ou un radical alkyle linéaire ou ramifié en C₁-C₆ (de préférence méthyle); Y désigne O ou NH; R₂ désigne un radical hydrocarboné hydrophobe comportant au moins de 6 à 50 atomes de carbone et plus-préférentiellement de 6 à 22 atomes de carbone et encore plus préférentiellement de 6 à 18 atomes de carbone et plus particulièrement de 12 à 16 atomes de carbone; x désigne un nombre de moles d'oxyde d'alkylène et varie de 0 à 100.

2. Composition selon la revendication 1, **caractérisée par le fait que** la partie hydrophobe du polymère amphiphile comporte de 6 à 22 atomes de carbone.

3. Composition selon la revendication 2, **caractérisée par le fait que** la partie hydrophobe du polymère amphiphile comporte de 6 à 18 atomes de carbone.

4. Composition selon la revendication 3, **caractérisée par le fait que** la partie hydrophobe du polymère amphiphile comporte de 12 à 18 atomes de carbone.

5. Composition selon l'une quelconque des revendications 1 à 4, **caractérisée par le fait que** les polymères amphiphiles sont neutralisés partiellement ou totalement par une base minérale ou organique.

6. Composition selon l'une quelconque des revendications 1 à 5, **caractérisée par le fait que** les polymères amphiphiles ont un poids moléculaire moyen en nombre allant de 1000 à 20 000 000 g/mole.

7. Composition selon la revendication 6, **caractérisée par le fait que** le poids moléculaire moyen en nombre varie de 20 000 à 5 000 000 g/mole.

8. Composition selon la revendication 7, **caractérisée par le fait que** le poids moléculaire moyen en nombre varie de 100 000 à 1 500 000 g/mole.

9. Composition selon l'une quelconque des revendications précédentes, **caractérisée par le fait qu'**une solution aqueuse à 1% en poids desdits polymères présente à la température de 25°C une viscosité mesurée au viscosimètre Brookfield, aiguille 7, allant de 20 000 mPas à 100 000 mPas.

10. Composition selon l'une quelconque des revendications précédentes, **caractérisée par le fait que** les polymères amphiphiles sont préparés par polymérisation radicalaire par précipitation dans le tert-butanol.

11. Composition selon l'une quelconque des revendications précedentes, **caractérisée par le fait que** les polymères amphiphiles sont réticulés ou non-réticulés.

12. Composition selon la revendication 11, **caractérisés par le fait que** les polymères amphiphiles sont réticulés.

13. Composition selon la revendication 12, **caractérisée par le fait que** le ou les agents de réticulation sont choisis parmi les composés à polyinsaturation oléfinique.

14. Composition selon la revendication 13, **caractérisée par le fait que** le ou les agents de réticulation sont choisis parmi le méthylène-bis-acrylamide, le méthacrylate d'allyle ou le triméthylol propane triacrylate (TMPTA).

15. Composition selon l'une quelconque des revendication 12 à 14, **caractérisée par le fait que** le taux de réticulation varie de préférence de 0,01 à 10% en moles et plus particulièrement de 0,2 à 2% en moles par rapport au polymère.

16. Composition selon la revendication 1, **caractérisée par le fait que** les polymères amphiphiles sont choisis parmi les polymères statistiques d'AMPS modifiés par réaction avec une n-mono(C₈-C₂₂)alkylamine ou une di-n-(C₈-C₂₂)alkylamine.

17. Composition selon la revendication 1, **caractérisée par le fait que** les polymères amphiphiles d'AMPS contiennent en plus au moins un monomère à insaturation éthylénique ne comportant pas de chaîne grasse.

18. Composition selon la revendication 17, **caractérisée par le fait que** le monomère à insaturation éthylénique ne comportant pas de chaîne grasse est choisi parmi les acides (méth)acryliques et leurs dérivés alkyl substitués en β, et leurs esters obtenus avec des monoalcools ou des mono- ou polyalkylèneglycols, ou bien parmi les (méth)acrylamides, la vinylpyrrolidone, l'anhydride maléique, l'acide itaconique ou l'acide maléique, ou les mélanges de ces composés.

19. Composition selon la revendication 1, **caractérisée par le fait que** le radical hydrophobe R₂ est choisi parmi les radicaux alkyles en C₆-C₁₈, linéaires, ramifiés ou cycliques; les radicaux alkylperfluorés en C₆-C₁₆ : le radical cholestéryle ou un ester de cholestérol; les groupes polycycliques aromatiques.

20. Composition salon l'une quelconque des revendications 1 ou 19 **caractérisée par le fait que** le monomère de formule (I) comporte en plus au moins un motif oxyde d'alkylène (x ≥ 1).

21. Composition selon l'une quelconque des revendications 1 ou 20, **caractérisée par le fait que** le monomère de formule (I) comporte en plus au moins une chaîne polyoxyalkylénée.

22. Composition selon la revendication 21, **caractérisée par le fait que** la chaîne polyoxyalkylénée est constituée de motifs oxyde d'éthylène et/ou de motifs oxyde de propylène.

23. Composition selon la revendication 22, **caractérisée par le fait que** la chaîne polyoxyalkylénée est constituée uniquement de motifs oxyde d'éthylène.

24. Composition selon l'une quelconque des revendications 1 ou 23, **caractérisée par le fait que** le nombre de motifs oxyalkylénés varie de 3 à 100.

25. Composition selon la revendication 24, **caractérisée par le fait que** le nombre de motifs oxyalkylénés varie de 3 à 50.

26. Composition selon la revendication 25, **caractérisée par le fait que** le nombre de motifs oxyalkylénés varie de 7 à 25.

27. Composition selon la revendication 1, **caractérisée par le fait que** le polymère amphiphile d'AMPS est choisi parmi:
- les copolymères réticulés ou non réticulés, neutralisés ou non, comportant de 15 à 60% en poids de motifs AMPS et de 40 à 85% en poids de motifs (C₈-C₁₈)alkyl(méth)acrylamide ou de motifs (C₈-C₁₈)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs n-(C₈₋₁₆)alkylacrylamide, par rapport au polymère.

28. Composition selon la revendication 27, **caractérisée par le fait que** le polymère amphiphile d'AMPS est choisi parmi :
- les copolymères non réticulés d'AMPS partiellement ou totalement neutralisé et de méthacrylate de n-dodécyle.
- les copolymères réticulés ou non réticulés d'AMPS partiellement ou totalement neutralisé et de n-dodécylméthacrylamide.

29. Composition selon la revendication 1, **caractérisée par le fait que** le polymère amphiphile d'AMPS est choisi parmi les copolymères constitués de motifs acide 2-acrylamido-2-méthylpropane-sulfonique (AMPS) de formule (II) suivante: 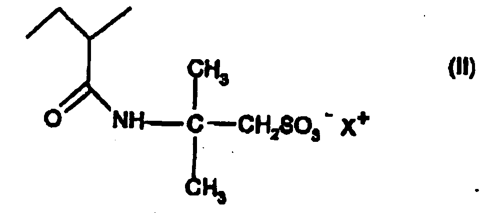 dans laquelle X⁺ est un proton, un cation de métal alcalin, un cation alcalino-terreux ou l'ion ammonium,
et de motifs de formule (III) suivante: 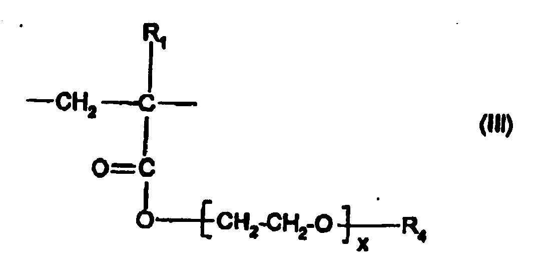 dans laquelle x désigne un nombre entier variant de 3 à 100, de préférence de 5 à 80 et plus préférentiellement de 7 à 25 ; R₁ a la même signification que celle indiquée ci-dessus dans la formule (I) et R₄ désigne un alkyle linéaire ou ramifié en C₆-C₂₂ et plus préférentiellement en C₁₀-C₂₂.

30. Composition selon la revendication 29, **caractérisée par le fait que** x = 25, R₁ est méthyle et R₄ est n-dodécyle.

31. Composition selon la revendication 1 ou 29, **caractérisée par le fait que** la proportion molaire en % des motifs de formule (I) ou des motifs de formule (III) dans les polymères varie de 50,1 à 99,9%.

32. Composition selon la revendication 1 ou 29, **caractérisée par le fait que** la proportion molaire en % des motifs de formule (I) ou des motifs de formule (III) dans les polymères varie de 0,1 à 50%.

33. Composition selon l'une quelconque des revendications précédentes, **caractérisée par le fait que** les polymères amphiphiles sont présents dans des concentrations allant de 0.01 à 30% en poids, plus préférentiellement de 0,1 à 10% en poids, encore plus préférentiellement de 0,1 à 5% en poids, et plus particulièrement encore de 0,5 à 2% en poids par rapport au poids total de la composition.

34. Composition selon l'une quelconque des revendications précédentes, **caractérisée par le fait que** le colorant d'oxydation est choisi parmi les bases d'oxydation et/ou les coupleurs.

35. Composition selon la revendication 34, **caractérisée par le fait qu'**elle contient au moins une base d'oxydation.

36. Composition selon les revendications 34 ou 35, **caractérisée par le fait que** les bases d'oxydation sont choisies parmi les ortho- ou para- phénylènediamines, les bases doubles, les ortho- ou para-, aminophénols, et les bases hétérocycliques, ainsi que les sels d'addition de ces composés avec un acide.

37. Composition selon la revendication 36, **caractérisée par** la fait que les para-phénylènediamines sont choisies parmi les composés de structure (I) suivante: 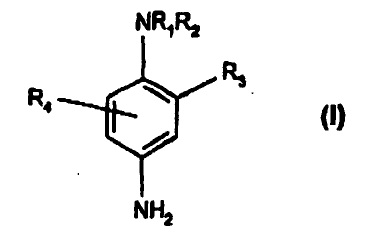 dans laquelle:
R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄.
R₄ représente un atome d'hydrogène, d'halogène ou un radical alkyle en C₁-C₄.

38. Composition selon la revendication 36, **caractérisée par le fait que** les bases doubles sont choisies parmi les composés de structure (II) suivante: 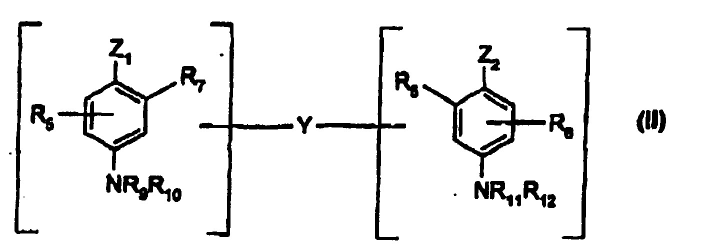 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₄;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaision Y;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

39. Composition selon la revendication 36, **caractérisée par le fait que** les paraaminophénols sont choisis parmi les composés de structure (III) suivante: 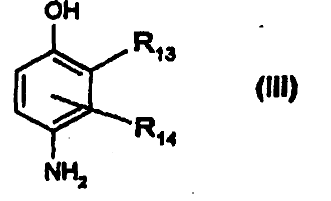 dans laquelle:
R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome dhydrogène ou un atome d'halogène tel que le fluor, un radical alkyle en C₁-C₄, monchydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

40. Composition selon la revendication 36, **caractérisée par le fait que** les bases hétérocycliques sont choisies parmi les dérivés pyridiniques, les dérivés pyrimidiniques dont les pyrazolopyrimidines, les dérivés pyrazoliques.

41. Composition selon l'une quelconque des revendications 36 à 40, **caractérisée par le fait que** les bases d'oxydation sont présentes dans des concentrations allant de 0,0005 à 12% en poids par rapport au poids total de la composition.

42. Composition selon la revendication 34, **caractérisée par le fait que** les coupleurs sont choisis parmi les métaphénylènediamines, les méta-aminophénols, les métadiphénols, les coupleurs hétérocycliques, et les sels d'addition de ces composés avec un acide.

43. Composition selon l'une quelconque des revendications 38 ou 42 **caractérisée par le fait que** les coupleurs sont présents dans des concentrations allant de 0.0001 à 10% en poids par rapport au poids total de la composition.

44. Composition selon l'une quelconque des revendications 34 à 43, **caractérisée par le fait que** les sels d'addition avec un acide des colorants d'oxydation sont choisis parmi les chlorohydrates, les bromhydrates, les sulfates, les tartrates, les lactates et les acétates.

45. Composition selon l'une quelconque des revendications précédentes, **caractérisée par le fait qu'**elle comprend en outre des colorants directs.

46. Composition selon l'une quelconque des revendications précédentes, **caractérisée par le fait qu'**elle comprend en outre au moins un polymère amphotère ou un polymère cationique.

47. Composition selon la revendication 46, **caractérisée par le fait que** le polymère cationique est un polyammonium quaternaire constitué de motifs récurrents répondant à la formule (W) suivante:

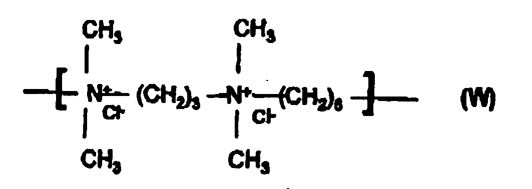

48. Composition selon la revendication 46, **caractérisée par le fait que** le polymère cationique est un polyammonium quaternaire constitué de motifs récurrents répondant à la formule (U) suivante :

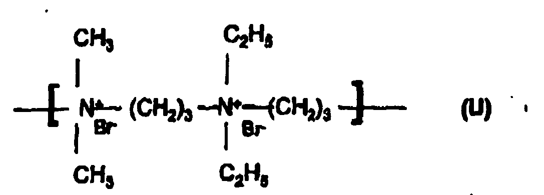

49. Composition selon la revendication 46, **caractérisée par le fait que** le polymère amphotère est un copolymère comprenant au moins comme monomères de l'acide acrylique et un sel de diméthyldiallylammonium.

50. Composition selon l'une quelconque des revendications 46 à 49, **caractérisée par le fait que** le ou les polymères cationiques ou amphotères représentent de 0,01 % à 10 %, de préférence de 0,05 % à 5 %, et encore plus préférentiellement de 0,1 % à 3 % en poids, du poids total de la composition.

51. Composition selon l'une quelconque des revendications précédentes, **caractérisée par le fait qu'**elle comprend au moins un tensioactif choisi parmi les tensioactifs anioniques, cationiques, non ioniques ou amphotères.

52. Composition selon la revendication 51, **caractérisée par le fait que** les tensioactifs représentent 0,01 à 40% et de préférence de 0,1 à 30% en poids, du poids total de la composition.

53. Composition selon l'une quelconque des revendications précédentes, **caractérisée par le fait qu'**elle comprend en outre au moins un agent réducteur, dans des quantités allant de 0,05 à 3% en poids par rapport au poids total de la composition.

54. Composition selon l'une quelconque des revendications précédentes, **caractérisée par le fait qu'**elle comprend en outre un agent oxydant.

55. Composition selon la revendication 54, par le fait que l'agent oxydant est choisi dans le groupe formé par le peroxyde d'hydrogène, le peroxyde d'urée, les bromates ou ferricyanures de métaux alcalins, les persels, les enzymes d'oxydoréduction avec éventuellement leur donneur ou cofacteur respectif.

56. Composition selon la revendication 55, **caractérisée par le fait que** l'agent oxydant est le peroxyde d'hydrogène.

57. Composition selon la revendication 56, **caractérisée par le fait que** l'agent oxydant est une solution d'eau oxygénée dont la titre varie de 1 à 40 volumes.

58. Composition selon la revendication 54, **caractérisée par** la fait qu'elle possède un pH allant de 4 à 11.

59. Procédé de teinture des fibres kératiniques et en particulier des fibres kératiniques humaines telles que les cheveux, **caractérisé par le fait qu'**il consiste à appliquer sur les fibres au moins une composition colorante comprenant, dans un milieu approprié pour la teinture, au moins un colorant d'oxydation, la couleur étant révélée à pH alcalin, neutre ou acide à l'aide d'une composition oxydante comprenant au moine un agent oxydant, qui est mélangée juste au moment de l'emploi à la composition colorante ou qui est appliquée séquentiellement sans rinçage intermédiaire, au moins un polymère amphiphile tel que défini à l'une quelconque des revendications 1 à 33 étant présent dans la composition colorante ou dans la composition oxydante ou dans chacune des deux compositions.

60. Procédé selon la revendication 59, **caractérisé par le fait qu'**il consiste à appliquer sur les fibres kératiniques sèches ou humides, la composition prête à l'emploi, réalisée extemporanément au moment de l'emploi à partir des compositions colorante et oxydante décrites ci-avant, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

61. Procédé de teinture des fibres kératiniques et en particulier des fibres kératiniques humaines telles que les cheveux, **caractérisé par** les fait qu'il consiste à appliquer sur les fibres kératiniques sèches ou humides, la composition prête à l'emploi, réalisée extemporanément au moment de l'emploi à partir d'une composition colorante comprenant au moins un colorant d'oxydation, d'une autre composition comprenant au moins un polymère amphiphile tel que défini selon les revendications 1 à 33, et d'une composition oxydante, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

62. Procédé selon la revendication 61, **caractérisé par le fait que** l'une ou l'autre ou lesdites compositions comprennent en outre au moins un polymère cationique ou amphotère et au moins un tensioactif.

63. Dispositif à 2 compartiments ou « Kit » pour la teinture des fibres kératiniques et en particulier des fibres kératiniques humaines telles que les cheveux, **caractérisé par le fait qu'**un compartiment renferme une composition colorante comprenant, dans un milieu approprié pour la teinture, au moins un colorant d'oxydation, et qu'un autre compartiment renferme une composition oxydante comprenant, dans un milieu approprié pour la teinture, un agent oxydant, au moins un polymère amphiphile tel que défini à rune quelconque des revendications 1-33 étant présent dans la composition colorante ou la composition oxydante, ou dans chacune des deux compositions.

64. Dispositif à 3 compartiments pour la teinture des fibres kératiniques et en particulier des fibres kératiniques humaines telles que les cheveux, **caractérisé par le fait qu'**un premier compartiment renferme une composition colorante comprenant, dans un milieu approprié pour la teinture, au moins un colorant d'oxydation, un deuxième compartiment renferme une composition oxydante comprenant, dans un milieu approprié pour la teinture au moins un agent oxydant, et un troisième compartiment renferme une composition comprenant, dans un milieu approprié pour la teinture, au moins un polymère amphiphile tel que défini à l'une quelconque des revendications 1-33, la composition colorante et/ou la composition oxydante pouvant également comprendre un polymère amphiphile tel que défini aux revendications 1-33.

## Claims

1. Oxidation dye composition for keratin fibres, in particular for human keratin fibres and more particularly the hair, comprising at least one oxidation dye in a medium that is suitable for dyeing, **characterized in that** it also comprises at least one amphiphilic polymer comprising at least one ethylenically unsaturated monomer containing a sulfonic group which is 2-acrylamido-2-methylpropanesulfonic acid, in free form or partially or totally neutralized form, and also at least one hydrophobic portion containing from 6 to 50 carbon atoms and corresponding to at least one ethylenically unsaturated hydrophobic monomer chosen from the acrylates or acrylamides of formula (I) below: 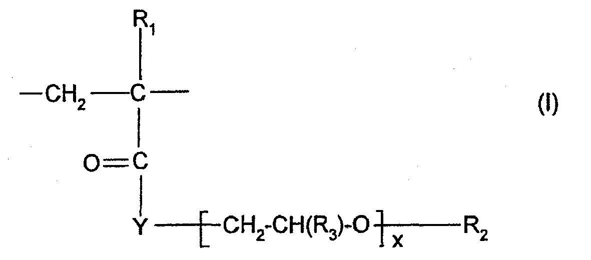 in which R₁ and R₃, which may be identical or different, denote a hydrogen atom or a linear or branched C₁-C₆ alkyl radical (preferably methyl); Y denotes O or NH; R₂ denotes a hydrophobic hydrocarbon-based radical containing at least from 6 to 50 carbon atoms, more preferably from 6 to 22 carbon atoms, even more preferably from 6 to 18 carbon atoms and more particularly from 12 to 18 carbon atoms; x denotes a number of moles of alkylene oxide and ranges from 0 to 100.

2. Composition according to Claim 1, **characterized in that** the hydrophobic portion of the amphiphilic polymer contains from 6 to 22 carbon atoms.

3. Composition according to Claim 2, **characterized in that** the hydrophobic portion of the amphiphilic polymer contains from 6 to 18 carbon atoms.

4. Composition according to Claim 3, **characterized in that** the hydrophobic portion of the amphiphilic polymer contains from 12 to 18 carbon atoms.

5. Composition according to any one of Claims 1 to 4, **characterized in that** the amphiphilic polymers are partially or totally neutralized with a mineral or organic base.

6. Composition according to any one of Claims 1 to 5, **characterized in that** the amphiphilic polymers have a number-average molecular weight ranging from 1000 to 20 000 000 g/mol.

7. Composition according to Claim 6, **characterized in that** the number-average molecular weight ranges from 20 000 to 5 000 000 g/mol.

8. Composition according to Claim 7, **characterized in that** the number-average molecular weight ranges from 100 000 to 1 500 000 g/mol.

9. Composition according to any one of the preceding claims, **characterized in that** an aqueous solution at 1% by weight of said polymers has, at a temperature of 25°C, a viscosity, measured using a Brookfield viscometer with a No. 7 needle, ranging from 20 000 mPa.s to 100 000 mPa.s.

10. Composition according to any one of the preceding claims, **characterized in that** the amphiphilic polymers are prepared by free-radical precipitation polymerization in tert-butanol.

11. Composition according to any one of the preceding claims, **characterized in that** the amphiphilic polymers are crosslinked or noncrosslinked.

12. Composition according to Claim 11, **characterized in that** the amphiphilic polymers are crosslinked.

13. Composition according to Claim 12, **characterized in that** the crosslinking agent(s) is (are) chosen from polyolefinically unsaturated compounds.

14. Composition according to Claim 13, **characterized in that** the crosslinking agent(s) is (are) chosen from methylenebisacrylamide, allyl methacrylate and trimethylolpropane triacrylate (TMPTA).

15. Composition according to any one of Claims 12 to 14, **characterized in that** the degree of crosslinking preferably ranges from 0.01 mol% to 10 mol% and more particularly from 0.2 mol% to 2 mol% relative to the polymer.

16. Composition according to Claim 1, **characterized in that** the amphiphilic polymers are chosen from random AMPS polymers modified by reaction with an n-mono (C₆-C₂₂) alkylamine or a di-n-(C₆-C₂₂) alkylamine.

17. Composition according to Claim 1, **characterized in that** the amphiphilic AMPS polymers also contain at least one ethylenically unsaturated monomer not comprising a fatty chain.

18. Composition according to Claim 17, **characterized in that** the ethylenically unsaturated monomer not comprising a fatty chain is chosen from (meth)acrylic acids and β-substituted alkyl derivatives thereof, and esters thereof obtained with monoalcohols or mono- or polyalkylene glycols, or alternatively from (meth)acrylamides, vinylpyrrolidone, maleic anhydride, itaconic acid or maleic acid, or mixtures of these compounds.

19. Composition according to Claim 1, **characterized in that** the hydrophobic radical R₂ is chosen from linear, branched or cyclic C₆-C₁₈ alkyl radicals; C₆-C₁₈ alkylperfluoro radicals; the cholesteryl radical or a cholesterol ester; aromatic polycyclic groups.

20. Composition according to either of Claims 1 and 19, **characterized in that** the monomer of formula (I) also comprises at least one alkylene oxide unit (x ≥ 1) .

21. Composition according to either of Claims 1 and 20, **characterized in that** the monomer of formula (I) also comprises at least one polyoxyalkylenated chain.

22. Composition according to Claim 21, **characterized in that** the polyoxyalkylenated chain consists of ethylene oxide units and/or of propylene oxide units.

23. Composition according to Claim 22, **characterized in that** the polyoxyalkylenated chain consists solely of ethylene oxide units.

24. Composition according to either of Claims 1 and 23, **characterized in that** the number of oxyalkylene units ranges from 3 to 100.

25. Composition according to Claim 24, **characterized in that** the number of oxyalkylene units ranges from 3 to 50.

26. Composition according to Claim 25, **characterized in that** the number of oxyalkylene units ranges from 7 to 25.

27. Composition according to Claim 1, **characterized in that** the amphiphilic AMPS polymer is chosen from:
- crosslinked or noncrosslinked, neutralized or nonneutralized copolymers comprising from 15% to 60% by weight of AMPS units and from 40% to 85% by weight of (C₈-C₁₆) alkyl (meth) acrylamide units or of (C₈-C₁₆) alkyl (meth) acrylate units relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units relative to the polymer.

28. Composition according to Claim 27, **characterized in that** the amphiphilic AMPS polymer is chosen from:
- noncrosslinked copolymers of partially or totally neutralized AMPS and of n-dodecyl methacrylate;
- crosslinked or noncrosslinked copolymers of partially or totally neutralized AMPS and of n-dodecylmethacrylamide.

29. Composition according to Claim 1, **characterized in that** the amphiphilic AMPS polymer is chosen from copolymers consisting of 2-acrylamido-2-methylpropanesulfonic acid (AMPS) units of formula (II) below: 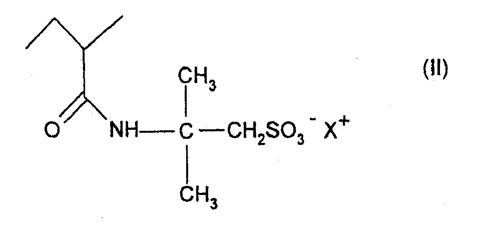 in which X⁺ is a proton, an alkali metal cation, an alkaline-earth metal cation or the ammonium ion, and of units of formula (III) below: 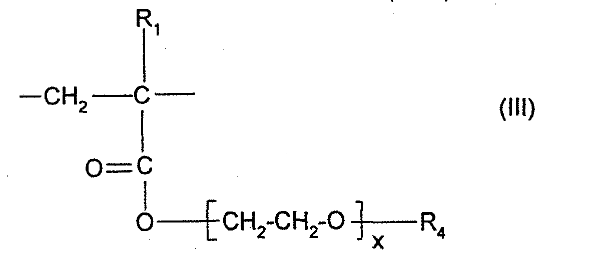 in which x denotes an integer ranging from 3 to 100, preferably from 5 to 80 and more preferably from 7 to 25; R₁ has the same meaning as that given above in formula (I) and R₄ denotes a linear or branched C₆-C₂₂ and more preferably C₁₀-C₂₂ alkyl.

30. Composition according to Claim 29, **characterized in that** x = 25, R₁ is methyl and R₄ is n-dodecyl.

31. Composition according to Claim 1 or 29, **characterized in that** the molar percentage proportion of units of formula (I) or of units of formula (III) in the polymers ranges from 50.1% to 99.9%.

32. Composition according to Claim 1 or 29, **characterized in that** the molar percentage proportion of units of formula (I) or of units of formula (III) in the polymers ranges from 0.1% to 50%.

33. Composition according to any one of the preceding claims, **characterized in that** the amphiphilic polymers are present in concentrations ranging from 0.01% to 30% by weight, more preferably from 0.1% to 10% by weight, even more preferably from 0.1% to 5% by weight and even more particularly from 0.5% to 2% by weight relative to the total weight of the composition.

34. Composition according to any one of the preceding claims, **characterized in that** the oxidation dye is chosen from oxidation bases and/or couplers.

35. Composition according to Claim 34, **characterized in that** it contains at least one oxidation base.

36. Composition according to Claim 34 or 35, **characterized in that** the oxidation bases are chosen from ortho- or para-phenylenediamines, double bases, ortho- or para-aminophenols and heterocyclic bases, and also the addition salts of these compounds with an acid.

37. Composition according to Claim 36, **characterized in that** the para-phenylenediamines are chosen from the compounds of structure (I) below: 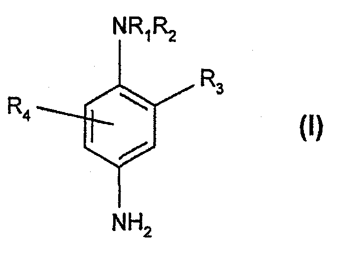 in which:
R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy(C₁-C₄)alkyl radical or a C₁-C₄ alkyl radical substituted with a nitrogenous, phenyl or 4'-aminophenyl group;
R₂ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous group;
R₁ and R₂ may also form, with the nitrogen atom that bears them, a 5- or 6-membered nitrogenous heterocycle optionally substituted with one or more alkyl, hydroxyl or ureido groups;
R₃ represents a hydrogen atom, a halogen atom such as a chlorine atom, a C₁-C₄ alkyl radical, a sulfo radical, a carboxy radical, a C₁-C₄ monohydroxyalkyl radical, a C₁-C₄ hydroxyalkoxy radical, an acetylamino(C₁-C₄)alkoxy radical, a mesylamino (C₁-C₄) alkoxy radical or a carbamoylamino(C₁-C₄)alkoxy radical,
R₄ represents a hydrogen or halogen atom or a C₁-C₄ alkyl radical.

38. Composition according to Claim 36, **characterized in that** the double bases are chosen from the compounds of structure (II) below: 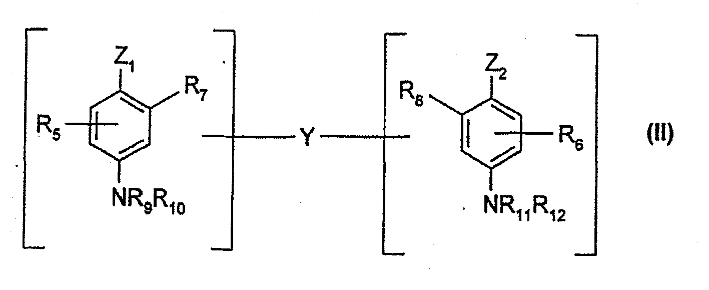 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more heteroatoms such as oxygen, sulfur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

39. Composition according to Claim 36, **characterized in that** the para-aminophenols are chosen from the compounds of structure (III) below: 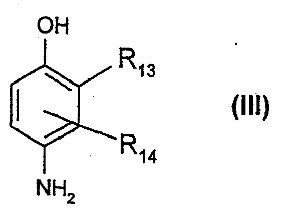 in which:
R₁₃ represents a hydrogen atom, a halogen atom, such as fluorine, or a C₁-C₄ alkyl, C₁-C₄ monohydroxyalkyl, (C₁-C₄) alkoxy(C₁-C₄) alkyl, C₁-C₄ aminoalkyl or hydroxy (C₁-C₄) alkylamino (C₁-C₄) alkyl radical,
R₁₄ represents a hydrogen atom, a halogen atom, such as fluorine, or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄)alkyl radical.

40. Composition according to Claim 36, **characterized in that** the heterocyclic bases are chosen from pyridine derivatives, pyrimidine derivatives, including pyrazolopyrimidines, and pyrazole derivatives.

41. Composition according to Claims 36 to 40, **characterized in that** the oxidation bases are present in concentrations ranging from 0.0005% to 12% by weight relative to the total weight of the composition.

42. Composition according to Claim 34, **characterized in that** the couplers are chosen from meta-phenylenediamines, meta-aminophenols, meta-diphenols and heterocyclic couplers, and the addition salts of these compounds with an acid.

43. Composition according to either of Claims 34 and 42, **characterized in that** the couplers are present in concentrations ranging from 0.0001% to 10% by weight relative to the total weight of the composition.

44. Composition according to any one of Claims 34 to 42, **characterized in that** the addition salts with an acid of the oxidation dyes are chosen from the hydrochlorides, hydrobromides, sulfates, tartrates, lactates and acetates.

45. Composition according to any one of the preceding claims, **characterized in that** it also comprises direct dyes.

46. Composition according to any one of the preceding claims, **characterized in that** it also comprises at least one amphoteric polymer or one cationic polymer.

47. Composition according to Claim 46, **characterized in that** the cationic polymer is a polyquaternary ammonium consisting of repeating units corresponding to formula (W) below:

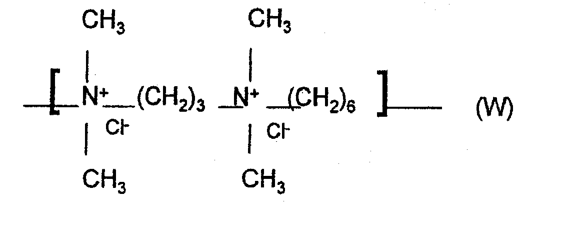

48. Composition according to Claim 46, **characterized in that** the cationic polymer is a polyquaternary ammonium consisting of repeating units corresponding to formula (U) below:

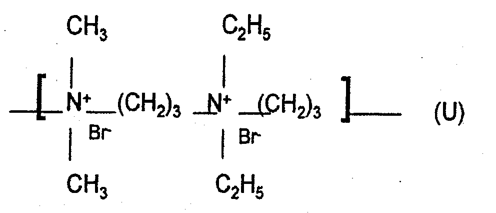

49. Composition according to Claim 46, **characterized in that** the amphoteric polymer is a copolymer comprising at least acrylic acid and a dimethyldiallylammonium salt as monomers.

50. Composition according to any one of Claims 46 to 49, **characterized in that** the cationic or amphoteric polymer(s) represent(s) from 0.01% to 10%, preferably from 0.05% to 5% and more preferably from 0.1% to 3% by weight relative to the total weight of the composition.

51. Composition according to any one of the preceding claims, **characterized in that** it comprises at least one surfactant chosen from anionic, cationic, nonionic and amphoteric surfactants.

52. Composition according to Claim 51, **characterized in that** the surfactants represent 0.01% to 40% and preferably 0.1% to 30% by weight relative to the total weight of the composition.

53. Composition according to any one of the preceding claims, **characterized in that** it also comprises at least one reducing agent in amounts ranging from 0.05% to 3% by weight relative to the total weight of the composition.

54. Composition according to any one of the preceding claims, **characterized in that** it also comprises an oxidizing agent.

55. Composition according to Claim 54, **characterized in that** the oxidizing agent is chosen from the group formed by hydrogen peroxide, urea peroxide, alkali metal bromates or ferricyanides, persalts, and redox enzymes optionally with the respective donor or cofactor thereof.

56. Composition according to Claim 55, **characterized in that** the oxidizing agent is hydrogen peroxide.

57. Composition according to Claim 56, **characterized in that** the oxidizing agent is an aqueous hydrogen peroxide solution with a titer ranging from 1 to 40 volumes.

58. Composition according to Claim 54, **characterized in that** it has a pH ranging from 4 to 11.

59. Process for dyeing keratin fibres, and in particular human keratin fibres such as the hair, **characterized in that** it consists in applying to the fibres at least one dye composition comprising at least one oxidation dye in a medium that is suitable for dyeing, the colour being developed at alkaline, neutral or acidic pH using an oxidizing composition comprising at least one oxidizing agent, which is mixed with the dye composition just at the time of use or which is applied sequentially without intermediate rinsing, at least one amphiphilic polymer as defined in any one of Claims 1 to 33 being present in the dye composition or in the oxidizing composition or in each of the two compositions.

60. Process according to Claim 59, **characterized in that** it consists in applying to the wet or dry keratin fibres the ready-to-use composition prepared extemporaneously at the time of use from the dye composition and oxidizing composition described above, leaving the composition to act for an action time ranging from 1 to 60 minutes approximately and preferably from 10 to 45 minutes, rinsing the fibres and then optionally washing them with shampoo, and then rinsing them again and drying them.

61. Process for dyeing keratin fibres, and in particular human keratin fibres such as the hair, **characterized in that** it consists in applying to the wet or dry keratin fibres the ready-to-use composition prepared extemporaneously at the time of use from a dye composition comprising at least one oxidation dye, another composition comprising at least one amphiphilic polymer as defined according to Claims 1 to 33, and an oxidizing composition, leaving the composition to act for an action time ranging from 1 to 60 minutes approximately and preferably from 10 to 45 minutes, rinsing the fibres and then optionally washing them with shampoo, and then rinsing them again and drying them.

62. Process according to Claim 61, **characterized in that** one or the other of said compositions also comprises at least one cationic or amphoteric polymer and at least one surfactant.

63. Two-compartment device or "kit" for dyeing keratin fibres, and in particular human keratin fibres such as the hair, **characterized in that** one compartment contains a dye composition comprising at least one oxidation dye in a medium that is suitable for dyeing, and another compartment contains an oxidizing composition comprising an oxidizing agent in a medium that is suitable for dyeing, at least one amphiphilic polymer as defined in any one of Claims 1 to 33 being present in the dye composition or in the oxidizing composition or in each of the two compositions.

64. Three-compartment device for dyeing keratin fibres, and in particular human keratin fibres such as the hair, **characterized in that** a first compartment contains a dye composition comprising at least one oxidation dye in a medium that is suitable for dyeing, a second compartment contains an oxidizing composition comprising at least one oxidizing agent in a medium that is suitable for dyeing, and a third compartment contains a composition comprising at least one amphiphilic polymer as defined in any one of Claims 1 to 33, in a medium that is suitable for dyeing, the dye composition and/or the oxidizing composition also possibly comprising an amphiphilic polymer as defined in Claims 1 to 33.

## Patentansprüche

1. Zusammensetzung zum oxidativen Färben von Keratinfasern und insbesondere menschlichen Keratinfasern, besonders zum Färben der Haare, die in einem zum Färben geeigneten Medium mindestens einen Oxidationsfarbstoff enthält, **dadurch gekennzeichnet, dass** sie ferner mindestens ein amphiphiles Polymer enthält, das mindestens ein Monomer mit ethylenisch ungesättigter Bindung und einer in freier Form oder ganz oder teilweise neutralisiert vorliegenden Sulfonsäuregruppe, bei dem es sich um 2-Acrylamido-2-methylpropansulfonsäure handelt, und ferner mindestens einen hydrophoben Bereich umfasst, der 6 bis 50 Kohlenstoffatome aufweist und mindestens einem hydrophoben Monomer mit ethylenisch ungesättigter Bindung entspricht, das unter den Acrylaten oder Acrylamiden der folgenden Formel (I) ausgewählt ist: 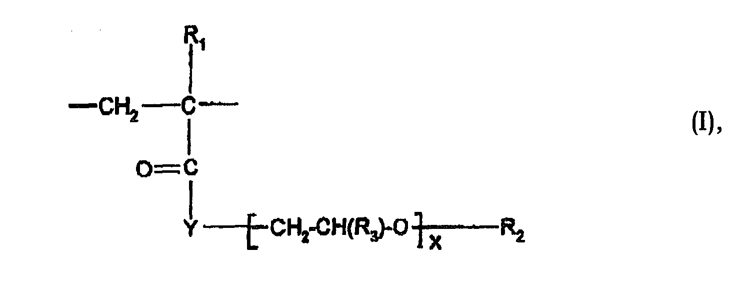 worin die Gruppen R₁ und R₃, die gleich oder verschieden sind, ein Wasserstoffatom oder eine geradkettige oder verzweigte C₁₋₆-Alkylgruppe (vorzugsweise Methyl) bedeuten; Y O oder NH bedeutet; R₂ ein hydrophobe Kohlenwasserstoffgruppe ist, die mindestens 6 bis 50 Kohlenstoffatome, bevorzugt 6 bis 22 Kohlenstoffatome, noch bevorzugter 6 bis 18 Kohlenstoffatome und insbesondere 12 bis 18 Kohlenstoffatome aufweist; und x die Molzahl von Alkylenoxid angibt und im Bereich von 0 bis 100 liegt.

2. Zusammensetzung nach Anspruch 1, **dadurch gekennzeichnet, dass** der hydrophobe Bereich des amphiphilen Polymers 6 bis 22 Kohlenstoffatom aufweist.

3. Zusammensetzung nach Anspruch 2, **dadurch gekennzeichnet, dass** der hydrophobe Bereich des amphiphilen Polymers 6 bis 18 Kohlenstoffatome aufweist.

4. Zusammensetzung nach Anspruch 3, **dadurch gekennzeichnet, dass** der hydrophobe Bereich des amphiphilen Polymers 12 bis 18 Kohlenstoffatome aufweist.

5. Zusammensetzung nach einem der Ansprüche 1 bis 4, **dadurch gekennzeichnet, dass** die amphiphilen Polymere ganz oder teilweise mit einer anorganischen oder organischen Base neutralisiert sind.

6. Zusammensetzung nach einem der Ansprüche 1 bis 5, **dadurch gekennzeichnet, dass** die amphiphilen Polymere eine zahlenmittlere Molmasse von 1 000 bis 20 000 000 g/mol aufweisen.

7. Zusammensetzung nach Anspruch 6, **dadurch gekennzeichnet, dass** die zahlenmittlere Molmasse im Bereich von 20 000 bis 5 000 000 g/mol liegt.

8. Zusammensetzung nach Anspruch 7, **dadurch gekennzeichnet, dass** die zahlenmittlere Molmasse im Bereich von 100 000 bis 1 500 000 g/mol liegt.

9. Zusammensetzung nach einem der vorhergehenden Ansprüche, **dadurch gekennzeichnet, dass** eine wässrige Lösung dieser Polymere von 1 Gew.-% bei einer Temperatur von 25 °C eine mit dem Brookfield-Viskosimeter, Nadel 7, gemessene Viskosität von 20 000 bis 100 000 mPa·s aufweist.

10. Zusammensetzung nach einem der vorhergehenden Ansprüche, **dadurch gekennzeichnet, dass** die amphiphilen Polymere durch radikalische Polymerisation unter Fällen in *t*-Butanol hergestellt werden.

11. Zusammensetzung nach einem der vorhergehenden Ansprüche, **dadurch gekennzeichnet, dass** die amphiphilen Polymere vernetzt oder nicht vernetzt sind.

12. Zusammensetzung nach Anspruch 11, **dadurch gekennzeichnet, dass** die amphiphilen Polymere vernetzt sind.

13. Zusammensetzung nach Anspruch 12, **dadurch gekennzeichnet, dass** das oder die Vernetzungsmittel unter den Verbindungen mit mehreren olefinisch ungesättigten Bindungen ausgewählt sind.

14. Zusammensetzung nach Anspruch 13, **dadurch gekennzeichnet, dass** das oder die Vernetzungsmittel unter Methylen-bis-acrylamid, Allylmethacrylat oder Trimethylolpropantriacrylat (TMPTA) ausgewählt sind.

15. Zusammensetzung nach einem der Ansprüche 12 bis 14, **dadurch gekennzeichnet, dass** der Vernetzungsgrad vorzugsweise im Bereich von 0,01 bis 10 Mol-% und insbesondere 0,2 bis 2 Mol-%, bezogen auf das Polymer, liegt.

16. Zusammensetzung nach Anspruch 1, **dadurch gekennzeichnet, dass** die amphiphilen Polymere unter den statistischen AMPS-Polymeren ausgewählt sind, die durch Umsetzung mit einem *n-*Mono(C₆₋₂₂)alkylamin oder einem Di-*n*-(C₆₋₂₂)alkylamin modifiziert wurden.

17. Zusammensetzung nach Anspruch 1, **dadurch gekennzeichnet, dass** die amphiphilen AMPS-Polymere ferner mindestens ein Monomer mit ethylenisch ungesättigter Bindung umfassen, das keine Fettkette aufweist.

18. Zusammensetzung nach Anspruch 17, **dadurch gekennzeichnet, dass** das Monomer mit ethylenisch ungesättigter Bindung, das keine Fettkette aufweist, unter (Meth)acrylsäure und ihren in β-Stellung substituierten Alkylderivaten und ihren Estern, die mit Monoalkoholen oder Mono- oder Polyalkylenglycolen gebildet werden, oder (Meth)acrylamiden, Vinylpyrrolidon, Maleinsäureanhydrid, Itaconsäure oder Maleinsäure oder den Gemischen dieser Verbindungen ausgewählt ist.

19. Zusammensetzung nach Anspruch 1, **dadurch gekennzeichnet, dass** die hydrophobe Gruppe R₂ unter den geradkettigen, verzweigten oder cyclischen C₆₋₁₈-Alkylgruppen; alkylperfluorierten Gruppen mit 6 bis 18 Kohlenstoffatomen; der Cholesterylgruppe oder einem Cholesterinester; aromatischen polycyclischen Gruppen ausgewählt ist.

20. Zusammensetzung nach einem der Ansprüche 1 bis 19, **dadurch gekennzeichnet, dass** das Monomer der Formel (I) ferner mindestens eine Alkylenoxideinheit aufweist (x ≥ 1).

21. Zusammensetzung nach einem der Ansprüche 1 oder 20, **dadurch gekennzeichnet, dass** das Monomer der Formel (I) ferner mindestens eine Polyoxyalkylenkette umfasst.

22. Zusammensetzung nach Anspruch 21, **dadurch gekennzeichnet, dass** die Polyoxyalkylenkette aus Ethylenoxideinheiten und/oder Propylenoxideinheiten besteht.

23. Zusammensetzung nach Anspruch 22, **dadurch gekennzeichnet, dass** die Polyoxyalkylenkette ausschließlich aus Ethylenoxideinheiten besteht.

24. Zusammensetzung nach einem der Ansprüche 1 oder 23, **dadurch gekennzeichnet, dass** die Anzahl der Oxyalkyleneinheiten im Bereich von 3 bis 100 liegt.

25. Zusammensetzung nach Anspruch 24, **dadurch gekennzeichnet, dass** die Anzahl der Oxyalkyleneinheiten im Bereich von 3 bis 50 liegt.

26. Zusammensetzung nach Anspruch 25, **dadurch gekennzeichnet, dass** die Anzahl der Oxyalkyleneinheiten im Bereich von 7 bis 25 liegt.

27. Zusammensetzung nach Anspruch 1, **dadurch gekennzeichnet, dass** das amphiphile AMPS-Polymer ausgewählt ist unter:
- vernetzten oder nicht vernetzten, neutralisierten oder nicht neutralisierten Copolymeren, die 15 bis 60 Gew.-% AMPS-Einheiten und 40 bis 85 Gew.-% (C₈₋₁₆)Alkyl(meth)acrylamideinheiten oder (C₈₋₁₆)Alkyl(meth)acrylateinheiten, bezogen auf das Polymer, enthalten;
- Terpolymeren, die 10 bis 90 Mol-% Acrylamideinheiten, 0,1 bis 10 Mol-% AMPS-Einheiten und 5 bis 80 Mol-% *n*-(C₆-₁₈)-Alkylacrylamideinheiten, bezogen auf das Polymer, enthalten.

28. Zusammensetzung nach Anspruch 27, **dadurch gekennzeichnet, dass** das amphiphile AMPS-Polymer ausgewählt ist unter:
- nicht vernetzten Copolymeren von ganz oder teilweise neutralisierter AMPS und n-Dodecylmethacrylat,
- vernetzten oder nicht vernetzten Copolymeren von ganz oder teilweise neutralisierter AMPS und n-Dodecylmethacrylamid.

29. Zusammensetzung nach Anspruch 1, **dadurch gekennzeichnet, dass** das amphiphile AMPS-Polymer unter den Copolymeren ausgewählt ist, die aus 2-Acrylamido-2-methylpropansulfonsäure-Einheiten (AMPS) der folgenden Formel (II): 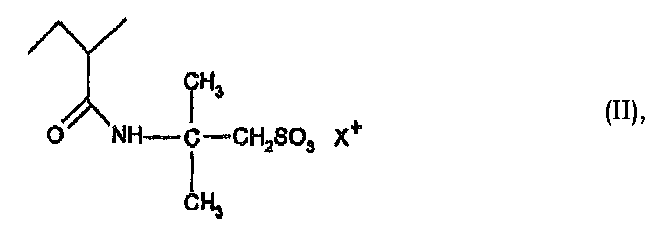 worin X⁺ ein Proton, ein Alkalimetallkation, ein Erdalkalimetallkation oder das Ammoniumion bedeutet,
und Einheiten der folgenden Formel (III) bestehen: 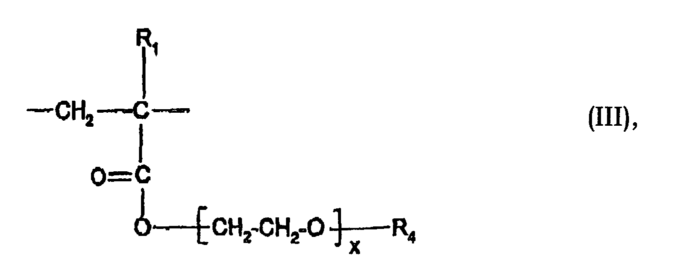 worin x eine ganze Zahl von 3 bis 100, vorzugsweise 5 bis 80 und noch bevorzugter 7 bis 25 ist; R₁ die oben für Formel (I) angegebenen Bedeutungen aufweist und R₄ eine geradkettige oder verzweigte Alkylgruppe mit 6 bis 22 Kohlenstoffatomen und insbesondere 10 bis 22 Kohlenstoffatomen ist.

30. Zusammensetzung nach Anspruch 29, **dadurch gekennzeichnet, dass** x = 25, R₁ bedeutet Methyl und R₄ bedeutet *n*-Dodecyl.

31. Zusammensetzung nach Anspruch 1 oder 29, **dadurch gekennzeichnet, dass** der prozentuale Molanteil der Einheiten der Formel (I) oder der Einheiten der Formel (III) in den Polymeren im Bereich von 50,1 bis 99,9 % liegt.

32. Zusammensetzung nach Anspruch 1 oder 29, **dadurch gekennzeichnet, dass** der prozentuale Molanteil der Einheiten der Formel (I) oder der Einheiten der Formel (III) in den Polymeren im Bereich 0, 1 bis 50 % liegt.

33. Zusammensetzung nach einem der vorhergehenden Ansprüche, **dadurch gekennzeichnet, dass** die amphiphilen Polymere in Konzentrationen von 0,01 bis 30 Gew.-%, noch bevorzugter 0,1 bis 10 Gew.-%, besonders bevorzugt 0,1 bis 5 Gew.-% und insbesondere 0,5 bis 2 Gew.-%, bezogen auf das Gesamtgewicht der Zusammensetzung, enthalten sind.

34. Zusammensetzung nach einem der vorhergehenden Ansprüche, **dadurch gekennzeichnet, dass** der Oxidationsfarbstoff unter den Oxidationsbasen und/oder Kupplern ausgewählt ist.

35. Zusammensetzung nach Anspruch 34, **dadurch gekennzeichnet, dass** sie mindestens eine Oxidationsbase enthält.

36. Zusammensetzung nach Anspruch 34 oder 35, **dadurch gekennzeichnet, dass** die Oxidationsbasen unter den *o*- oder *p*-Phenylendiaminen, Doppelbasen, o- oder *p*-Aminophenolen und heterocyclischen Basen sowie den Additionssalzen dieser Verbindungen mit einer Säure ausgewählt sind.

37. Zusammensetzung nach Anspruch 36, **dadurch gekennzeichnet, dass** die *p*-Phenylendiamine unter den Verbindungen der folgenden Struktur (I) ausgewählt sind: 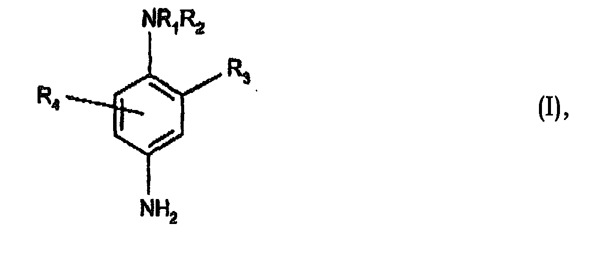 worin bedeuten:
R₁ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), eine mit einer stickstoffhaltigen Gruppe substituierte C₁₋₄-Alkylgruppe, Phenyl oder 4'-Aminophenyl;
R₂ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄) oder mit einer stickstoffhaltigen Gruppe substituierte C₁₋₄-Alkylgruppe;
R₁ und R₂ können auch mit dem Stickstoffatom, das sie trägt, einen 5- oder 6-gliedrigen Stickstoffheterocyclus bilden, der gegebenenfalls mit einer oder mehreren Gruppen Alkyl, Hydroxy oder Ureido substituiert ist;
R₃ ein Wasserstoffatom, ein Halogenatom, wie ein Chloratom, C₁₋₄-Alkyl, Sulfo, Carboxy, C₁₋₄-Monohydroxyalkyl, C₁₋₄-Hydroxyalkoxy, C₁₋₄-Acetylaminoalkoxy, C₁₋₄-Mesylaminoalkoxy oder C₁₋₄-Carbamoylaminoalkoxy;
R₄ ein Wasserstoffatom, ein Halogenatom oder C₁₋₄-Alkyl.

38. Zusammensetzung nach Anspruch 36, **dadurch gekennzeichnet, dass** die Doppelbasen unter den Verbindungen der folgenden Struktur (II) ausgewählt sind: 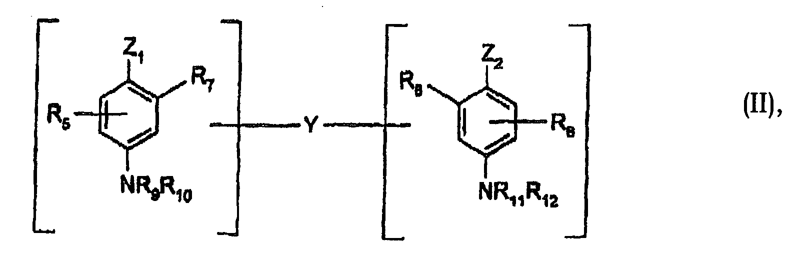 worin bedeuten:
- Z₁ und Z₂, die gleich oder verschieden sind, eine Gruppe Hydroxy oder -NH₂, die mit einer C₁₋₄-Alkylgruppe oder einer Verbindungsgruppe Y substituiert sein kann;
- die Verbindungsgruppe Y eine geradkettige oder verzweigte Alkylenkette mit 1 bis 14 Kohlenstoffatomen, die durch eine oder mehrere stickstoffhaltige Gruppen und/oder durch ein oder mehrere Heteroatome, wie Sauerstoff, Schwefel oder Stickstoff, unterbrochen oder abgeschlossen werden kann und gegebenenfalls mit einer oder mehreren Hydroxygruppen oder C₁₋₆-Alkoxygruppen substituiert ist;
- R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y;
- R₇, R₈, R₉, Rio, R₁₁ und R₁₂, die gleich oder verschieden sind, ein Wasserstoffatom, eine Verbindungsgruppe Y oder C₁₋₄-Alkyl;
mit der Maßgabe, dass die Verbindungen der Formel (II) nur eine Verbindungsgruppe Y pro Molekül aufweisen.

39. Zusammensetzung nach Anspruch 36, **dadurch gekennzeichnet, dass** die p-Aminophenole unter den Verbindungen der folgenden Struktur (III) ausgewählt sind: 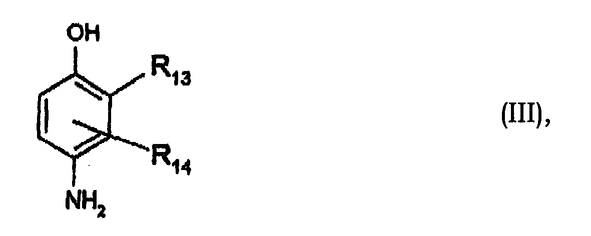 worin bedeuten:
R₁₃ ein Wasserstoffatom, ein Halogenatom, wie Fluor, C₁₋₄-Alkyl, C₁₋₄-Monohydroxyalkyl, Alkoxy(C₁₋₄)alkyl(C₁₋₄), C₁₋₄-Aminoalkyl oder Hydroxyalkyl(C₁₋₄)aminoalkyl(C₁₋₄),
R₁₄ ein Wasserstoffatom, ein Halogenatom, wie Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder Alkoxy(C₁₋₄)alkyl(C₁₋₄).

40. Zusammensetzung nach Anspruch 36, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

41. Zusammensetzung nach einem der Ansprüche 36 bis 40, **dadurch gekennzeichnet, dass** die Oxidationsbasen in Konzentrationen von 0,0005 bis 12 Gew.-%, bezogen auf das Gesamtgewicht der Zusammensetzung, vorliegen.

42. Zusammensetzung nach Anspruch 34, **dadurch gekennzeichnet, dass** die Kuppler unter *m*-Phenylendiaminen, *m*-Aminophenolen, *m*-Dihydroxybenzolen, heterocyclischen Kupplern und den Additionssalzen dieser Verbindungen mit einer Säure ausgewählt sind.

43. Zusammensetzung nach einem der Ansprüche 34 oder 42, **dadurch gekennzeichnet, dass** die Kuppler in Konzentrationen von 0,0001 bis 10 Gew.-%, bezogen auf das Gesamtgewicht der Zusammensetzung, enthalten sind.

44. Zusammensetzung nach einem der Ansprüche 34 bis 43, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

45. Zusammensetzung nach einem der vorhergehenden Ansprüche, **dadurch gekennzeichnet, dass** sie ferner Direktfarbstoffe enthält.

46. Zusammensetzung nach einem der vorhergehenden Ansprüche, **dadurch gekennzeichnet, dass** sie ferner mindestens ein amphoteres Polymer oder ein kationisches Polymer enthält.

47. Zusammensetzung nach Anspruch 46, **dadurch gekennzeichnet, dass** das kationische Polymer ein quartäres Polyammonium ist, das aus Wiederholungseinheiten der folgenden Formel (W) besteht:

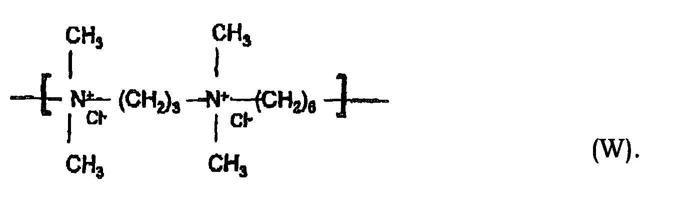

48. Zusammensetzung nach Anspruch 46, **dadurch gekennzeichnet, dass** das kationische Polymer ein quartäres Polyammonium ist, das aus Wiederholungseinheiten der folgenden Formel (U) besteht:

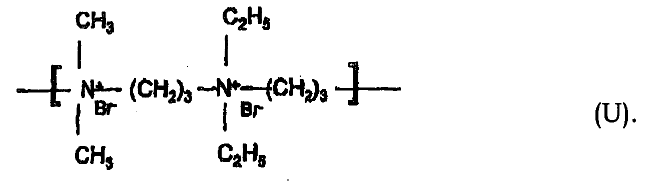

49. Zusammensetzung nach Anspruch 46, **dadurch gekennzeichnet, dass** das amphotere Polymer ein Copolymer ist, das als Monomere zumindest Acrylsäure und ein Dimethyldiallylammoniumsalz enthält.

50. Zusammensetzung nach einem der Ansprüche 46 bis 49, **dadurch gekennzeichnet, dass** das oder die kationischen oder amphoteren Polymere 0,01 bis 10 %, vorzugsweise 0,05 bis 5 % und noch bevorzugter 0,1 bis 3 Gew.-% des Gesamtgewichts der Zusammensetzung ausmachen.

51. Zusammensetzung nach einem der vorhergehenden Ansprüche, **dadurch gekennzeichnet, dass** sie zumindest einen grenzflächenaktiven Stoff enthält, der unter den anionischen, kationischen, nichtionischen oder amphoteren grenzflächenaktiven Stoffen ausgewählt ist.

52. Zusammensetzung nach Anspruch 51, **dadurch gekennzeichnet, dass** die grenzflächenaktiven Stoffe 0,01 bis 40 Gew.-% und vorzugweise 0,1 bis 30 Gew.-% des Gesamtgewichts der Zusammensetzung ausmachen.

53. Zusammensetzung nach einem der vorhergehenden Ansprüche, **dadurch gekennzeichnet, dass** sie ferner mindestens ein Reduktionsmittel in Mengenanteilen von 0,05 bis 3 Gew.-%, bezogen auf das Gesamtgewicht der Zusammensetzung, enthält.

54. Zusammensetzung nach einem der vorhergehenden Ansprüche, **dadurch gekennzeichnet, dass** sie ferner ein Oxidationsmittel enthält.

55. Zusammensetzung nach Anspruch 54, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen gegebenenfalls zusammen mit ihrem jeweiligen Donor oder Cofaktor ausgewählt ist.

56. Zusammensetzung nach Anspruch 55, **dadurch gekennzeichnet, dass** es sich bei dem Oxidationsmittel um Wasserstoffperoxid handelt.

57. Zusammensetzung nach Anspruch 56, **dadurch gekennzeichnet, dass** das Oxidationsmittel eine Wasserstoffperoxidlösung ist, deren Titer im Bereich von 1 bis 40 Volumina liegt.

58. Zusammensetzung nach Anspruch 54, **dadurch gekennzeichnet, dass** sie einen pH-Wert von 4 bis 11 aufweist.

59. Verfahren zum Färben von Keratinfasern und insbesondere menschlichen Keratinfasern, wie Haaren, **dadurch gekennzeichnet, dass** es darin besteht, auf die Fasern mindestens eine Farbmittelzusammensetzung aufzutragen, die in einem zum Färben geeigneten Medium mindestens einen Oxidationsfarbstoff enthält, wobei die Farbe bei einem alkalischen, neutralen oder sauren pH-Wert mit einer oxidierenden Zusammensetzung gebildet wird, die mindestens ein Oxidationsmittel enthält und die bei der Anwendung mit der Farbmittelzusammensetzung vermischt wird oder die ohne zwischenzeitliches Spülen anschließend aufgetragen wird, wobei mindestens ein amphiphiles Polymer, wie es in einem der Ansprüche 1 bis 33 definiert ist, in der Farbmittelzusammensetzung oder in der oxidierenden Zusammensetzung oder in beiden Zusammensetzungen enthalten ist.

60. Verfahren nach Anspruch 59, **dadurch gekennzeichnet, dass** es darin besteht, auf die trockenen oder feuchten Keratinfasern die gebrauchsfertige Zusammensetzung aufzutragen, die bedarfsgemäß bei der Anwendung aus der Farbmittelzusammensetzung und der oxidierenden Zusammensetzung, die oben beschrieben wurden, gebildet wird, diese während einer Einwirkzeit von etwa 1 bis 60 Minuten und vorzugsweise 10 bis 45 Minuten einwirken zu lassen, die Fasern zu spülen, gegebenenfalls mit Haarwaschmittel zu waschen und nochmals zu spülen und sie zu trocknen.

61. Verfahren zum Färben von Keratinfasern und insbesondere menschlichen Keratinfasern, wie Haaren, **dadurch gekennzeichnet, dass** es darin besteht, auf die trockenen oder feuchten Keratinfasern die gebrauchsfertige Zusammensetzung aufzutragen, die bedarfsgemäß bei der Anwendung aus einer Farbmittelzusammensetzung, die mindestens einen Oxidationsfarbstoff enthält, einer weiteren Zusammensetzung, die mindestens ein amphiphiles Polymer enthält, wie es in einem der Ansprüche 1 bis 33 definiert wurde, und einer oxidierenden Zusammensetzung gebildet wird, diese während einer Einwirkzeit von etwa 1 bis 60 Minuten und vorzugsweise 10 bis 45 Minuten einwirken zu lassen, die Fasern zu spülen, gegebenenfalls mit Haarwaschmittel zu waschen und anschließend nochmals zu spülen und die Fasern zu trocknen.

62. Verfahren nach Anspruch 61, **dadurch gekennzeichnet, dass** eine der beiden Zusammensetzungen oder beide Zusammensetzungen ferner mindestens ein kationisches oder amphoteres Polymer und mindestens einen grenzflächenaktiven Stoff enthalten.

63. Vorrichtung mit zwei Abteilungen oder «Kit» zum Färben von Keratinfasern und insbesondere menschlichen Keratinfasern wie Haaren, **dadurch gekennzeichnet, dass** eine Abteilung eine Farbmittelzusammensetzung enthält, die in einem zum Färben geeigneten Medium mindestens einen Oxidationsfarbstoff enthält, und eine andere Abteilung eine oxidierende Zusammensetzung enthält, die in einem zum Färben geeigneten Medium ein Oxidationsmittel enthält, wobei mindestens ein amphiphiles Polymer, wie es in einem der Ansprüche 1 bis 33 definiert wurde, in der Farbmittelzusammensetzung oder in der oxidierenden Zusammensetzung oder in beiden Zusammensetzungen enthalten ist.

64. Vorrichtung mit drei Abteilung zum Färben von Keratinfasern und insbesondere menschlichen Keratinfasern, wie Haaren, **dadurch gekennzeichnet, dass** eine erste Abteilung eine Farbmittelzusammensetzung enthält, die in einem zum Färben geeigneten Medium mindestens einen Oxidationsfarbstoff enthält, eine zweite Abteilung eine oxidierende Zusammensetzung enthält, die in einem zum Färben geeigneten Medium mindestens ein Oxidationsmittel enthält, und eine dritte Abteilung eine Zusammensetzung enthält, die in einem zum Färben geeigneten Medium mindestens ein amphiphiles Polymer, wie es in einem der Ansprüche 1 bis 33 definiert wurde, enthält, wobei die Farbmittelzusammensetzung und/oder die oxidierende Zusammensetzung auch ein amphiphiles Polymer nach einem der Ansprüche 1 bis 33 enthalten können.
